# EUROPEAN PATENT APPLICATION

(11) **EP 3 970 896 A1**
(43) Date of publication of application: **23.03.2022**
(21) Application number: 20196623.1
(22) Date of filing: 17.09.2020
(51) Int. Cl.: B23K 9/32, A61F 9/06

(54) **ANTI-GLARE WELDING APPARATUS**

(71) Applicant: ARCMASK OPTECH CO., LTD, Taoyuan City (TW)
(72) Inventor: HSIEH, Chien Hsing, Taoyuan City (TW); CHEN, Chia Hung, Taoyuan City (TW)
(74) Representative: Viering, Jentschura & Partner mbB Patent- und Rechtsanwälte

(57) **Abstract**

An anti-glare welding apparatus includes a switch (10) and a controller (20). The controller (20) is electrically connected to the switch (10). When the controller (20) determines that the switch (10) is switched from an OFF state to an ON state, the controller (20) generates a welding helmet turn-on signal to switch on a filter of a welding helmet (50). The controller (20) further generates a welding machine turn-on signal after a first delay time to switch on a welding machine (40). The filter of the welding helmet (50) can be fully darkened during the first delay time when receiving the welding helmet turn-on signal, and the welding machine (40) is switched on after the filter of the welding helmet (50) is fully darkened. Therefore, there is no welding arc when the filter is darkening, and the eyes of the welding worker can be fully protected from damage.

## Description

### 1. Field of the Invention

The present invention relates to a welding device, and more particularly to an anti-glare welding apparatus.

### 2. Description of the Related Art

A welding worker needs to wear a welding helmet during work. The welding helmet can protect the eyes, face, or neck of the welding worker from flash burn, ultraviolet light, sparks, infrared light, and heat.

A common welding helmet includes a dark filter, and the welding worker can clearly see a welding position through the dark filter. The dark filter can block a welding arc to protect the eyes of the welding worker. However, when the welding worker is not working, the welding worker cannot clearly see his surroundings with the welding helmet. Hence, the welding worker has to take off the welding helmet for clearly seeing his surroundings when he is not working.

Further, another common welding helmet includes a filter which can automatically adjust transmittance of the filter. During welding, the welding arc is generated, and the filter can automatically darken to lower the transmittance of the filter. When the welding worker is not welding, no welding arc is generated, and the filter can automatically brighten to raise the transmittance of the filter. Therefore, the welding worker does not need to take off the welding helmet and can clearly see his surroundings through the brightened filter when he is not welding.

However, the welding helmet having the filter which can automatically adjust transmittance of the filter automatically adjusts the transmittance of the filter according to whether the welding arc is generated or not. When the welding helmet detects that the welding arc is generated, the welding helmet needs a time period to darken the filter. Namely, the filter is not darkened at the time that the welding arc is generated. Since the filter of the welding helmet cannot immediately darken the filter, the welding arc still can pass through the filter of the welding helmet when the filter is darkening to damage the eyes of the welding worker. Therefore, the welding helmet needs to be further improved.

An objective of the present invention is to provide an anti-glare welding apparatus which can fully protect eyes of a welding worker.

The anti-glare welding apparatus includes a switch and a controller.

The switch has an ON state and an OFF state.

The controller is electrically connected to the switch. When the controller determines that the switch is switched from the OFF state to the ON state, the controller generates a welding helmet turn-on signal, and wirelessly transmits the welding helmet turn-on signal. The controller further generates a welding machine turn-on signal after a first delay time, and transmits the welding machine turn-on signal.

When a welding worker presses the switch for welding, the controller determines that the switch is switched from the OFF state to the ON state. The controller firstly generates and wirelessly transmits the welding helmet turn-on signal for controlling a filter of a welding helmet to darken, and after the first delay time, the controller secondly generates the welding machine turn-on signal for switching on a welding machine.

The filter of the welding helmet can be fully darkened during the first delay time. The welding machine is switched on after the filter of the welding helmet is fully darkened, and the welding worker can weld to generate a welding arc. Namely, the welding arc is generated after the filter of the welding helmet is fully darkened. Therefore, there is no welding arc when the filter is darkening, and the eyes of the welding worker can be fully protected from damage.

Other objectives, advantages, and novel features of the invention will become more apparent from the following detailed description when taken in conjunction with the accompanying drawings.

### IN THE DRAWINGS:

Fig. 1 is a block diagram of an embodiment of the present invention;
Fig. 2 is a schematic view of a welding gun of the present invention;
Fig. 3 is a schematic view of a welding helmet of the present invention;
Fig. 4 shows waveform diagrams of the present invention;
Fig. 5 is a block diagram of the welding helmet of the present invention.

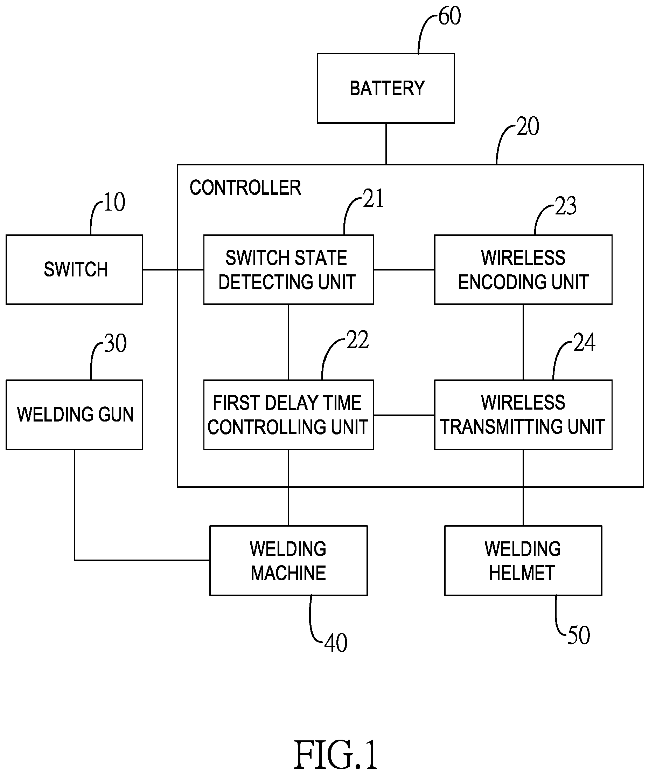

With reference to Fig. 1, the present invention is an anti-glare welding apparatus. An embodiment of the anti-glare welding apparatus include a switch 10 and a controller 20. The switch 10 has an ON state and an OFF state. The controller 20 is electrically connected to the switch 10. When the controller 20 determines that the switch 10 is switched from the OFF state to the ON state, the controller 20 generates a welding helmet turn-on signal, and wirelessly transmits the welding helmet turn-on signal. The controller 20 further generates a welding machine turn-on signal after a first delay time, and transmits the welding machine turn-on signal.

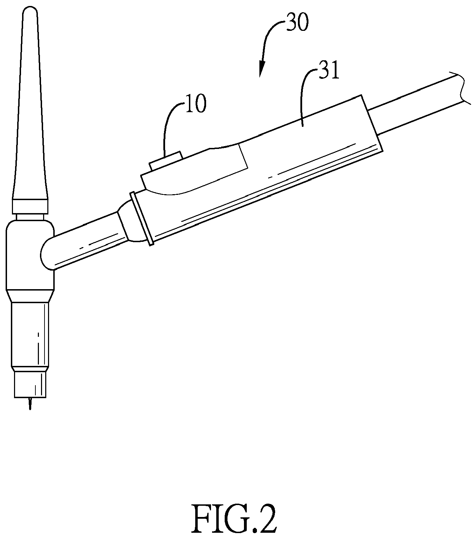

The anti-glare welding apparatus further includes a welding gun 30, a welding machine 40, and a welding helmet 50. With reference to Fig. 2, the switch 10 is mounted on a shell 31 of the welding gun 30. The welding machine 40 is electrically connected to the welding gun 30 and the controller 20. When the welding machine 40 receives the welding machine turn-on signal, the welding machine 40 powers the welding gun 30.

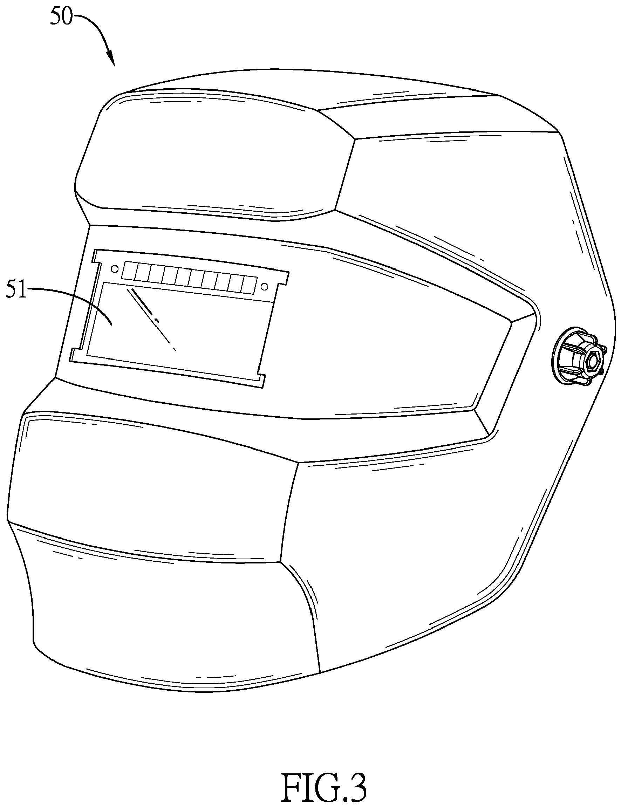

With reference to Fig. 3, the welding helmet 50 includes a liquid-crystal filter 51, and is wirelessly connected to the controller 20. When the welding helmet 50 wirelessly receives the welding helmet turn-on signal from the controller 20, the welding helmet 50 controls the liquid-crystal filter 51 to become dark for lowering a transmittance of the liquid-crystal filter 51.

For example, when the switch 10 is not pressed, the switch 10 is in the OFF state, the welding machine 40 does not power the welding gun 30, the welding worker cannot weld, and no welding arc is generated. The welding helmet 50 does not receive the welding helmet turn-on signal, and the liquid-crystal filter 51 does not darken. Therefore, the welding worker can clearly see his surroundings through the liquid-crystal filter 51.

To do the welding, the welding worker presses the switch 10, and the switch 10 is switched from the OFF state to the ON state. Therefore, the controller 20 can determine that the switch 10 is switched from the OFF state to the ON state, generates the welding helmet turn-on signal, and wirelessly transmits the welding helmet turn-on signal to the welding helmet 50. When the welding helmet 50 receives the welding helmet turn-on signal from the controller 20, the welding helmet 50 controls the liquid-crystal filter 51 to become dark for lowering a transmittance of the liquid-crystal filter 51. Therefore, the darkened liquid-crystal filter 51 can protect eyes of the welding worker from damage.

Moreover, when the controller 20 determines that the switch 10 is switched from the OFF state to the ON state, the controller 20 firstly generates the welding helmet turn-on signal, and after the first delay time, the controller 20 secondly generates the welding machine turn-on signal. Further, the controller 20 transmits the welding machine turn-on signal to the welding machine 40. When the welding machine 40 receives the welding machine turn-on signal, the welding machine 40 powers the welding gun 30, and the welding worker can start welding.

After generating the welding helmet turn-on signal after the first delay time, the welding machine turn-on signal is generated. Namely, after the welding worker presses the switch 10 after the first delay time, the welding machine is turned on to allow the welding worker to do the welding. However, when the welding worker presses the switch 10, the welding helmet turn-on signal is already transmitted to the welding helmet 50 to darken the liquid-crystal filter 51. The liquid-crystal filter 51 can fully darken during the first delay time. Therefore, when the welding machine is tuned on to allow the welding worker to do the welding, the liquid-crystal filter 51 is fully darkened. The welding arc cannot pass through the darkened liquid-crystal filter 51, and the eyes of the welding worker can be protected.

Moreover, in the embodiment, the controller 20 includes a switch state detecting unit 21, a first delay time controlling unit 22, a wireless encoding unit 23, and a wireless transmitting unit 24.

The switch state detecting unit 21 is electrically connected to the switch 10 to detect that the switch is in the ON state or the OFF state. When the switch state detecting unit 21 detects that the switch 10 is switched from the OFF state to the ON state, the switch state detecting unit 21 generates a turn-on signal.

The first delay time controlling unit 22 is electrically connected to the switch state detecting unit 21. When the first delay time controlling unit 22 receives the turn-on signal generated by the switch state detecting unit 21, the first delay time controlling unit 22 generates and transmits the welding machine turn-on signal to the welding machine 40 after the first delay time.

The wireless encoding unit 23 is electrically connected to the switch state detecting unit 21. When the wireless encoding unit 23 receives the turn-on signal generated by the switch state detecting unit 21, the wireless encoding unit 23 encodes the turn-on signal to the welding helmet turn-on signal. The wireless transmitting unit 24 is electrically connected to the wireless encoding unit 23, receives the welding helmet turn-on signal from the wireless encoding unit 23, and transmits the welding helmet turn-on signal to the welding helmet 50.

Moreover, when the controller 20 determines that the switch 10 is switched from the ON state to the OFF state, the controller 20 generates a welding helmet turn-off signal, and wirelessly transmits the welding helmet turn-off signal to the welding helmet 50. In the meantime, the controller 20 further generates a welding machine turn-off signal, and transmits the welding machine turn-off signal to the welding machine 40.

When the welding machine 40 receives the welding turn-off signal, the welding machine 40 is turned off, and the welding machine 40 stops powering the welding gun 30. Further, when the welding helmet 50 wirelessly receives the welding helmet turn-off signal, the welding helmet 50 controls the liquid-crystal filter 51 to become bright for increasing the transmittance of the liquid-crystal filter 51 after a second delay time.

For example, when the welding worker presses the switch 10, the switch 10 is in the ON state, the welding machine 40 is powering the welding gun 30, the welding worker can weld, and the liquid-crystal filter 51 is darkened. Therefore, the eyes of the welding worker can be protected.

When the welding worker releases the switch 10, the switch 10 is switched from the ON state to the OFF state. Therefore, the controller 20 determines that the switch 10 is switched from the ON state to the OFF state, generates the welding helmet turn-off signal, and wirelessly transmits the welding helmet turn-off signal to the welding helmet 50. When the welding helmet 50 receives the welding helmet turn-off signal from the controller 20, the welding helmet 50 firstly waits for the second delay time, and the welding helmet 50 secondly controls the liquid-crystal filter 51 to become bright for increasing the transmittance of the liquid-crystal filter 51. Then, the welding worker can clearly see his surroundings through the brightened liquid-crystal filter 51.

Moreover, when the controller 20 determines that the switch 10 is switched from the ON state to the OFF state, the controller 20 further generates the welding machine turn-off signal, and transmits the welding machine turn-off signal to the welding machine 40. Then, the welding machine 40 is turned off, and stops powering the welding gun 30. The welding worker stops welding.

The welding machine turned-off signal and the welding helmet turned-off signal are generated simultaneously. Namely, when the welding worker releases the switch 10, the welding machine 40 stops powering to the welding gun 30 immediately, and the welding worker stops welding. However, when the welding worker releases the switch 10, the welding helmet turn-off signal is wirelessly transmitted to the welding helmet 50 immediately. But, when the welding helmet 50 receives the welding helmet turn-off signal, the welding helmet 50 does not control the liquid-crystal filter 51 to become bright immediately. The welding helmet 50 waits for the second delay time, and then the welding helmet 50 controls the liquid-crystal filter 51 to become bright for increasing the transmittance of the liquid-crystal filter 51. Therefore, at the moment that the welding machine 40 stops powering the welding gun 30 and during the second delay time, the welding helmet 50 does not control the liquid-crystal filter 51 to become bright. The welding arc cannot pass through the liquid-crystal filter 51, and the eyes of the welding worker can be protected. After the welding helmet 50 wirelessly receives the welding helmet turn-off signal for the second delay time, the welding helmet 50 controls the liquid-crystal filter 51 to become bright for increasing the transmittance of the liquid-crystal filter 51. Then, the welding worker can clearly see his surroundings through the brightened liquid-crystal filter 51.

In the embodiment, when the switch state detecting unit 21 detects that the switch 10 is switched from the ON state to the OFF state, the switch state detecting unit 21 generates a turn-off signal.

When the first delay time controlling unit 22 receives the turn-off signal generated by the switch state detecting unit 21, the first delay time controlling unit 22 immediately generates and transmits the welding machine turn-off signal to the welding machine 40.

When the wireless encoding unit 23 receives the turn-off signal generated by the switch state detecting unit 21, the wireless encoding unit 23 encodes the turn-off signal to the welding helmet turn-off signal. The wireless transmitting unit 24 receives the welding helmet turn-off signal from the wireless encoding unit 23, and transmits the welding helmet turn-off signal to the welding helmet 50.

Moreover, the anti-glare welding apparatus further includes a battery 60. The battery 60 is electrically connected to the controller 20 to power the controller 20.

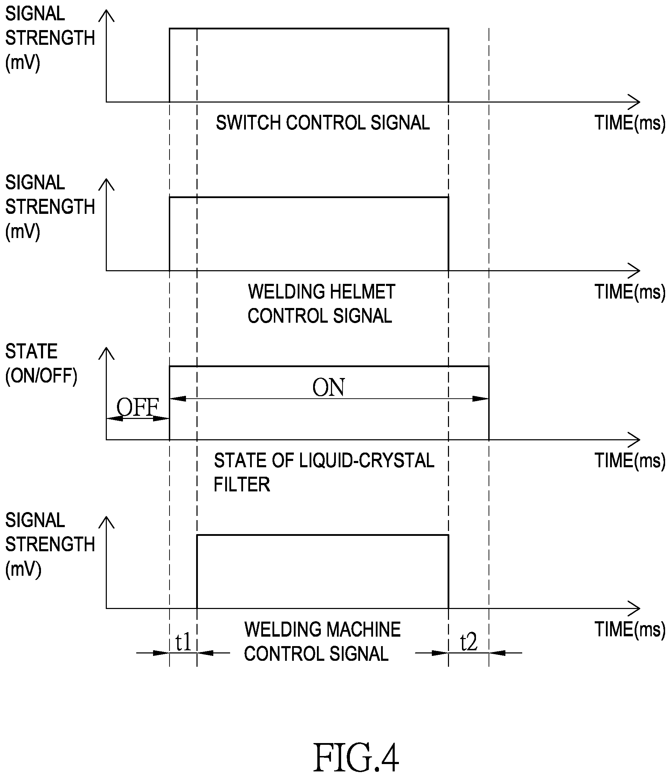

With reference to Fig. 4, when the controller 20 detects that the switch 10 is switched from the OFF state to the ON state, such as a raising edge of a waveform of a switch control signal, the controller 20 may generate the welding helmet turn-on signal, such as a raising edge of a waveform of a welding helmet control signal. When the welding helmet 50 receives the welding helmet turn-on signal, the welding helmet 50 controls a state of the liquid-crystal filter 51 from an OFF state to an ON state to make the liquid-crystal filter 51 become dark, such as a raising edge of a waveform of the state of the liquid-crystal filter 51. However, when the controller 20 generates the welding helmet turn-on signal, the welding machine turn-on signal may be generated after the first delay time t1, and the welding machine 50 may power the welding gun 30, such as a raising edge of a waveform of a welding machine control signal.

However, when the controller 20 detects that the switch 10 is switched from the ON state to the OFF state, such as a falling edge of the waveform of the switch control signal, the controller 20 may generate the welding helmet turn-off signal and the welding machine turn-off signal, such as a falling edge of the waveform of the welding helmet control signal and a falling edge of the waveform of the welding machine control signal. The welding machine 40 stops powering the welding gun 30 since the welding machine 40 receives the welding machine turn-off signal.

Further, when the welding helmet 50 receives the welding helmet turn-off signal, the welding helmet 50 controls a state of the liquid-crystal filter 51 from an ON state to an OFF state to make the liquid-crystal filter 51 become bright after the second delay time t2, such as a falling edge of the waveform of the state of the liquid-crystal filter 51.

For example, the first delay time t1 is greater than 0.05 seconds, but not limited thereto, and the second delay time t2 is greater than 0.01 seconds, but not limited thereto.

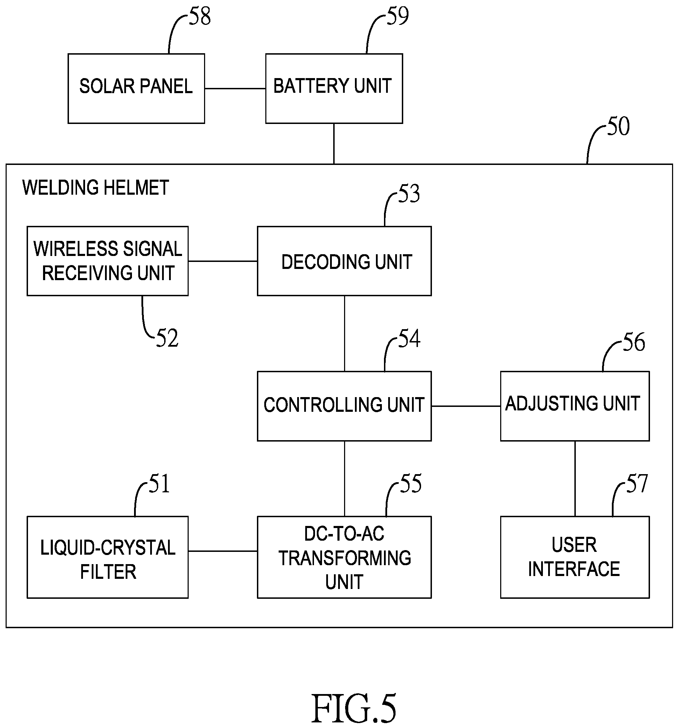

Further, with reference to Fig. 5, the welding helmet 50 includes the liquid-crystal filter 51, a wireless signal receiving unit 52, a decoding unit 53, a controlling unit 54, a DC-to-AC transforming unit 55, an adjusting unit 56, a user interface 57, a solar panel 58, and a battery unit 59.

The decoding unit 53 is electrically connected to the wireless signal receiving unit 52 and the controlling unit 54. The wireless signal receiving unit 52 wirelessly receives the welding helmet turn-on signal or the welding helmet turn-off signal. The decoding unit 53 receives the welding helmet turn-on signal or the welding helmet turn-off signal through the wireless signal receiving unit 52, decodes the welding helmet turn-on signal or the welding helmet turn-off signal, and transmits the decoded welding helmet turn-on signal or the decoded welding helmet turn-off signal to the controlling unit 54. The controlling unit 54 is electrically connected to the decoding unit 53 and the adjusting unit 56, and the user interface 57 is electrically connected to the adjusting unit 56. The user interface 57 is provided for the welding worker to adjust the color of the fully darkened liquid-crystal filter 51. The adjusting unit 56 generates an adjusting signal according to a state of the user interface 57, and transmits the adjusting signal to the controlling unit 54. The controlling unit 54 generates a corresponding controlling signal through the DC-to-AC transforming unit 55 to control the color of the liquid-crystal filter 51 according to the adjusting signal, the decoded welding helmet turn-on signal, or the decoded welding helmet turn-off signal.

The solar panel 58 is electrically connected to the battery unit 59 to receive solar power, and transforms the solar power to electric power for charging the battery unit 59. The battery unit 59 powers the liquid-crystal filter 51, the wireless signal receiving unit 52, the decoding unit 53, the controlling unit 54, the DC-to-AC transforming unit 55, the adjusting unit 56, and the user interface 57.

Even though numerous characteristics and advantages of the present invention have been set forth in the foregoing description, together with details of the structure and function of the invention, the disclosure is illustrative only. Changes may be made in detail, especially in matters of shape, size, and arrangement of parts within the principles of the invention to the full extent indicated by the broad general meaning of the terms in which the appended claims are expressed.

## Claims

1. An anti-glare welding apparatus, **characterized in that** the anti-glare welding apparatus comprises:
a switch (10), having an ON state and an OFF state; and
a controller (20), electrically connected to the switch (10);
wherein when the controller (20) determines that the switch (10) is switched from the OFF state to the ON state, the controller (20) generates a welding helmet (50) turn-on signal, and wirelessly transmits the welding helmet (50) turn-on signal; and
wherein when the controller (20) determines that the switch (10) is switched from the OFF state to the ON state, the controller (20) further generates a welding machine (40) turn-on signal after a first delay time, and transmits the welding machine (40) turn-on signal.

2. The anti-glare welding apparatus as claimed in claim 1, further comprising:
a welding gun (30); wherein the switch (10) is mounted on a shell (31) of the welding gun (30); and
a welding machine (40), electrically connected to the welding gun (30) and the controller (20); wherein when the welding machine (40) receives the welding machine (40) turn-on signal, the welding machine (40) powers the welding gun (30).

3. The anti-glare welding apparatus as claimed in claim 1, further comprising:
a welding helmet (50), having a liquid-crystal filter (51), and wirelessly connected to the controller (20);
wherein when the welding helmet (50) wirelessly receives the welding helmet (50) turn-on signal from the controller (20), the welding helmet (50) controls the liquid-crystal filter (51) to become dark for lowering a transmittance of the liquid-crystal filter (51).

4. The anti-glare welding apparatus as claimed in claim 1, wherein the controller (20) comprises:
a switch state detecting unit (21), electrically connected to the switch (10) to detect that the switch (10) is in the ON state or the OFF state; wherein when the switch state detecting unit (21) detects that the switch (10) is switched from the OFF state to the ON state, the switch state detecting unit (21) generates a turn-on signal;
a first delay time controlling unit (22), electrically connected to the switch state detecting unit (21); wherein when the first delay time controlling unit (22) receives the turn-on signal generated by the switch state detecting unit (21), the first delay time controlling unit (22) generates and transmits the welding machine (40) turn-on signal after the first delay time;
a wireless encoding unit (23), electrically connected to the switch state detecting unit (21); wherein when the wireless encoding unit (23) receives the turn-on signal generated by the switch state detecting unit (21), the wireless encoding unit (23) encodes the turn-on signal to the welding helmet (50) turn-on signal; and
a wireless transmitting unit (24), electrically connected to the wireless encoding unit (23); wherein the wireless transmitting unit (24) receives the welding helmet (50) turn-on signal from the wireless encoding unit (23), and transmits the welding helmet (50) turn-on signal.

5. The anti-glare welding apparatus as claimed in claim 1, further comprising:
a battery, electrically connected to the controller (20) to power the controller (20).

6. The anti-glare welding apparatus as claimed in claim 1, wherein when the controller (20) determines that the switch (10) is switched from the ON state to the OFF state, the controller (20) generates a welding helmet (50) turn-off signal, and wirelessly transmits the welding helmet (50) turn-off signal;
wherein when the controller (20) determines that the switch (10) is switched from the ON state to the OFF state, the controller (20) further generates a welding machine (40) turn-off signal, and transmits the welding machine (40) turn-off signal.

7. The anti-glare welding apparatus as claimed in claim 6, further comprising:
a welding gun (30); wherein the switch (10) is mounted on a shell (31) of the welding gun (30); and
a welding machine (40), electrically connected to the welding gun (30) and the controller (20); wherein when the welding machine (40) receives the welding machine (40) turn-off signal, the welding machine (40) stops powering the welding gun (30).

8. The anti-glare welding apparatus as claimed in claim 6, further comprising:
a welding helmet (50), having a liquid-crystal filter (51), and wirelessly connected to the controller (20);
wherein when the welding helmet (50) wirelessly receives the welding helmet (50) turn-off signal, the welding helmet (50) controls the liquid-crystal filter (51) to become bright for increasing the transmittance of the liquid-crystal filter (51) after a second delay time.

9. The anti-glare welding apparatus as claimed in claim 6, wherein the controller (20) comprises:
a switch state detecting unit (21), electrically connected to the switch (10) to detect that the switch (10) is in the ON state or the OFF state; wherein when the switch state detecting unit (21) detects that the switch (10) is switched from the ON state to the OFF state, the switch state detecting unit (21) generates a turn-off signal;
a first delay time controlling unit (22), electrically connected to the switch state detecting unit (21); wherein when the first delay time controlling unit (22) receives the turn-off signal generated by the switch state detecting unit (21), the first delay time controlling unit (22) generates and transmits the welding machine (40) turn-off signal;
a wireless encoding unit (23), electrically connected to the switch state detecting unit (21); wherein when the wireless encoding unit (23) receives the turn-off signal generated by the switch state detecting unit (21), the wireless encoding unit (23) encodes the turn-off signal to the welding helmet (50) turn-off signal; and
a wireless transmitting unit (24), electrically connected to the wireless encoding unit (23); wherein the wireless transmitting unit (24) receives the welding helmet (50) turn-off signal from the wireless encoding unit (23), and transmits the welding helmet (50) turn-off signal.
